# EUROPEAN PATENT SPECIFICATION

(11) **EP 2 961 393 B1**
(45) Date of publication and mention of the grant of the patent: **28.10.2020**
(21) Application number: 14710647.0
(22) Date of filing: 27.02.2014
(51) Int. Cl.: A61K 9/20, A61K 9/50, A61K 9/00, A61K 31/445, A61P 25/28, A61K 9/28, A61P 25/20, A61P 43/00

(54) **PHARMACEUTICAL COMPOSITIONS OF DONEPEZIL HAVING SPECIFIC IN VITRO DISSOLUTION PROFILE OR PHARMACOKINETICS PARAMETERS**
PHARMAZEUTISCHE ZUSAMMENSETZUNGEN VON DONEPEZIL MIT SPEZIFISCHEM IN-VITRO-AUFLÖSUNGSPROFIL ODER PHARMAKOKINETISCHEN PARAMETERN
COMPOSITIONS PHARMACEUTIQUES DE DONÉPÉZIL PRÉSENTANT UN PROFIL DE DISSOLUTION IN VITRO OU DES PARAMÈTRES PHARMACOCINÉTIQUES SPÉCIFIQUES

(30) Priority: 28.02.2013 IN 991KO2012
(43) Date of publication of application: 06.01.2016
(73) Proprietor: Lupin Limited, Mumbai 400 055 (IN)
(72) Inventor: BAKHLE, Dhananjay, Sadashiv, Maharashtra Pune 412 115 (IN); SHAH, Chirag, Anilkumar, Maharashtra Pune 412 115 (IN); GADVE, Snehal, Ameet, Maharashtra Pune 412 115 (IN); SHARMA, Neha, Maharashtra Pune 412 115 (IN); CHANDRAN, Sajeev, Maharashtra Pune 412 115 (IN); DESHMUKH, Ashish, Ashokrao, Maharashtra Pune 412 115 (IN)
(74) Representative: J A Kemp LLP
(86) International application number: PCT/IB2014/059302
(87) International publication number: WO 2014/132218

(56) References cited:
- WO-A1-00/30446
- WO-A1-2012/035409
- WO-A2-2011/107750
- US-A1- 2003 082 230
- US-A1- 2006 252 788
- US-A1- 2006 280 789
- US-A1- 2007 129 402
- Center for Drug Evaluation and Research: "Application Number 022568; Medical Review(s)", , 23 July 2010 (2010-07-23), page 1-94, XP002725060, Retrieved from the Internet: URL:http://www.accessdata.fda.gov/drugsatf da_docs/nda/2010/022568orig1s000medr.pdf [retrieved on 2014-05-15]
- MARTIN FARLOW ET AL: "Safety and tolerability of donepezil 23 mg in moderate to severe Alzheimer's disease", BMC NEUROLOGY, BIOMED CENTRAL LTD., LONDON, GB, vol. 11, no. 1, 25 May 2011 (2011-05-25), page 57, XP021099938, ISSN: 1471-2377, DOI: 10.1186/1471-2377-11-57
- GAUTHIER S: "Cholinergic adverse effects of cholinesterase inhibitors in Alzheimer's disease: epidemiology and management", DRUGS & AGING, ADIS INTERNATIONAL LTD, NZ, vol. 18, no. 11, 1 January 2001 (2001-01-01), pages 853-862, XP009136420, ISSN: 1170-229X
- JACKSON STEPHEN ET AL: "The safety and tolerability of donepezil in patients with Alzheimer's disease", British Journal of Clinical Pharmacology, vol. 58 Suppl 1 November 2004 (2004-11), pages 1-8, XP055120544, ISSN: 0306-5251 Retrieved from the Internet: URL:http://www.ncbi.nlm.nih.gov/pmc/articl es/PMC1884556/pdf/bcp0058-0001.pdf [retrieved on 2014-05-21]
- SINGER M ET AL: "Nightmares in patients with Alzheimer's disease caused by donepezil ; Therapeutic effect depends on the time of intake", DER NERVENARZT ; ORGAN DER DEUTSCHEN GESELLSCHAFT FÜR PSYCHIATRIE, PSYCHOTHERAPIE UND NERVENHEILKUNDE ORGAN DER DEUTSCHEN GESELLSCHAFT FÜR NEUROLOGIE, SPRINGER, BERLIN, DE, vol. 76, no. 9, 1 September 2005 (2005-09-01), pages 1127-1129, XP019321231, ISSN: 1433-0407, DOI: 10.1007/S00115-004-1856-7

## Description

### Field of the Invention

The present invention relates to a timed release pharmaceutical composition of donepezil wherein the composition provides a plasma donepezil concentration profile that corresponds with the circadian rhythm of the acetylcholine (Ach) levels in the brain. The composition reduces the incidence of sleep disturbances which include difficulty in falling asleep, multiple awakenings during sleep, nightmares, disrupted sleep-wake rhythms, and early morning awakenings and also reduce the incidence of sundowning effect observed in subjects suffering from Alzheimer's disease. The composition, upon oral administration provides reduced incidence of gastrointestinal (GI) side effects.

### Background

Donepezil, known chemically as (±)-2, 3-dihydro-5, 6-dimethoxy2-[[1-(phenylmethyl)-4-piperidinyl]methyl]-1H-inden-1-one, is a reversible inhibitor of the enzyme acetylcholinesterase. It is currently available in tablet dosage forms of 5mg, 10mg and 23mg doses under the trade name Aricept® or Aricep® or as orally disintegrating tablets (Aricept® ODT). Aricept® 23 mg film-coated tablet is a sustained-release formulation. Donepezil is indicated for the treatment of dementia of the Alzheimer's type with efficacy established in mild, moderate and severe Alzheimer's disease.

Alzheimer's disease (AD) is an age-related neurodegenerative disorder that is characterized by progressive loss of memory and deterioration of higher cognitive functions. In addition to its traditional symptomatology of deficits in cognitive function and memory, AD is characterized by a marked alteration in circadian rhythmicity, beyond that which is associated with ageing. In AD patients, the sleep-wake cycle is more disrupted than in healthy individuals and this can exacerbate behavioral disorders and memory dysfunction.

The sleep-wake cycle comprises three main phases: wakefulness, non-rapid eye movement (NREM) sleep and rapid eye movement (REM) sleep. On the basis of the depth of sleep, NREM sleep is subdivided into stages. In healthy young adults, sleep usually begins at the lightest stage, NREM stage 1, and progresses through stage 2 and into the deepest sleep states, stage 3 and stage 4, also known as delta or slow-wave sleep. The sleeper then progresses back to lighter sleep and then into REM sleep. In healthy individuals, a typical 8-hour period of sleep contains four or five cycles of these alternating sleep phases, with occasional brief awakenings. REM sleep accounts for 20-25% of total sleep time in most human adults.

It is well known that nightmares occur during the REM phase of sleep. The existing donepezil treatment increases the REM sleep percentage and hence causes nightmares.

Sleep disturbances are common in persons with AD. These sleep disturbances include difficulty in falling asleep, multiple awakenings during sleep, disrupted sleep-wake rhythms, and early morning awakenings. Sleep disturbances in persons with AD are a physical and psychological burden for their caregivers and are a major reason why such patients are admitted to long-term care institutions.

ACh plays an important role in maintaining a normal sleep pattern, which is important for memory consolidation. In the brain, ACh is thought to be released from cholinergic neurons according to a circadian rhythm. The cholinergic system is regulated for increased transmission during waking and motor activity and decreased transmission, in general, during sleep, with brief localized increases during REM sleep. The elements of the cholinergic system- synaptic ACh, stored ACh, acetylcholinesterase activity and cholinergic receptors- are coordinated to achieve this end. These rhythms may deteriorate with ageing and is a particular problem in AD.

Behavioral disturbances, such as nocturnal delirium, agitation or wandering are noticeable in older adults with AD and are believed to be associated with disrupted biological rhythms. This phenomenon is called "sundowning" which is a constellation of increasing behavioral disturbances in patients with dementia in the late afternoon or early evening. Sundowning represents an understudied area and the neurobiological basis of this behavioral pattern remains unspecified. One study demonstrates that this behavioral pattern coincides with time-dependent changes in basal forebrain acetylcholinesterase expression.

There exists a need to develop compositions of donepezil which provides reduced incidence of sleep disturbances, which include difficulty in falling asleep, multiple awakenings during sleep, disrupted sleep-wake rhythms and early morning awakenings. There also exists a need todevelop compositions of donepezil whichreduces the incidence of sundowning. These objectives can be achieved by formulating compositions which release donepezil in a pattern which corresponds with the circadian rhythm of the acetylcholine release from the cholinergic neurons.

The immediate release composition of donepezil results in a spike in the subject's blood plasma levels within 2 to 5 hours after administration of the drug. Following oral dosing, peak plasma concentration is achieved for Aricept® or Aricep® 10mg, in approximately 3 hours. This initial spike in blood plasma levels causes undesirable side effects in subjects, such as anxiety, nightmares, insomnia, and/or gastrointestinal problems such as nausea, vomiting and diarrhea.

Prior art discloses sustained release compositions as a solution for reducing the incidence of these side effects.

U.S. Patent Application 2005/0232990 discloses a sustained release formulation comprising amorphous donepezil or an amorphous pharmaceutically acceptable salt thereof and a pharmaceutically acceptable polymeric carrier, wherein the polymeric carrier maintains the active agent in substantially amorphous form.

U.S. Patent Applications 2006/0280789, 2007/0129402, 2006/0159753, 2009/0208579, and 2011/0045074 disclose orally administrable matrix type sustained release formulations comprising a basic drug, an enteric polymer, and, optionally, one or more compounds selected from water-insoluble polymers, water-soluble sugars, sugar alcohols, and pharmaceutically acceptable excipients.

U.S. Patent Applications 2008/0213368 and 2010/0152164 disclose pharmaceutical compositions containing an anti-dementia drug and sustained-release base, with storage stability of the anti-dementia drug, wherein a high molecular weight acidic substance is added for stabilizing the anti-dementia drug.

PCT Application WO 2011/069076 discloses a sustained release tablet formulation comprising donepezil or a pharmaceutically acceptable salt thereof, further comprising at least one release rate controlling material that is a hydrophilic material, hydrophobic material, enteric polymer, or any combination thereof.

U.S. Patent Application 2011/0218216 discloses an extended-release pharmaceutical composition for an oral administration comprising donepezil or pharmaceutically acceptable salt thereof, a release-controlling agent and a microenvironment pH modifier.

U.S. Patent Application 2011/0237623 discloses a sustained-release formulation for an acetylcholinesterase inhibitor, comprising an acetylcholinesterase inhibitor and at least two gel-forming polymers.

US 2006/280,789 and its continuation-in-part US 2007/129,402 describe sustained release formulations comprising donepezil, stereoisomers of donepezil, pharmaceutically acceptable salts of donepezil, or pharmaceutically acceptable salts of stereoisomers of donepezil. The formulations are said to have desirable pharmacokinetic characteristics such as AUC, Tmax, Cmax, dosage-corrected AUC, and dosage-corrected Cmax.

However, the label of Aricept® or Aricep® discloses that the gastrointestinal side effects (nausea, vomiting and diarrhea), when they occur, appear more frequently with the 10 mg/day dose than with the 5 mg/day dose and more frequently with the 23 mg dose (sustained release formulation) than with the 10 mg dose. Specifically, in a controlled trial that compared a dose of 23 mg/day to 10 mg/day in patients who had been treated with donepezil 10 mg/day for at least three months, the incidence of nausea in the 23 mg group was markedly greater than in the patients who continued on 10 mg/day (11.8% vs. 3.4%, respectively), and the incidence of vomiting in the 23 mg group was markedly greater than in the 10 mg group (9.2% vs. 2.5%, respectively). The percent of patients who discontinued treatment due to vomiting in the 23 mg group was markedly higher than in the 10 mg group (2.9% vs. 0.4%, respectively).

Public Citizen, representing more than 225,000 members and supporters nationwide, had petitioned to the US FDA in Jun 2011 to immediately remove the 23 mg dose of Aricept® from the market because the 23 mg dose of Aricept® failed to meet the two efficacy criteria required by FDA as a condition of approval of drugs for dementia, and because the 23 mg dose of Aricept® significantly increased adverse events compared to the previously approved 10 mg dose, including increased risks for nausea, vomiting, diarrhea, anorexia, and confusion.

Hence, there is still a need for developing compositions of donepezil which provide a reduced incidence of undesirable gastrointestinal side effects in subjects, which include nausea, vomiting and diarrhea.

### Summary of the Invention

The invention provides a timed release pharmaceutical composition of donepezil, which can provide a plasma donepezil concentration profile that corresponds with the circadian rhythm of the ACh levels in the brain. The timed release pharmaceutical composition of the invention comprises donepezil or a pharmaceutically acceptable salt thereof, wherein the composition is a coated composition, which comprises:
a) a core comprising donepezil in an amount from 3 mg to 50 mg, and one or more release controlling agent(s), and
b) a functional coating comprising one or more release controlling agent(s); wherein the release controlling agent(s) is/are selected from hydrophilic release controlling agents, hydrophobic release controlling agents, natural release controlling agents and synthetic release controlling agents;
the amount of release controlling agent in the composition is 10% w/w to 50% w/w by total weight of the composition; and
the composition exhibits the following in vitro dissolution profile when tested in a Paddle dissolution apparatus at 50 rpm in 900 ml pH 6.8 buffer at 37°C less than 20% w/w of donepezil is released in 3-4 hrs, and more than 85% w/w of donepezil is released after 12 hrs.

The composition can provide reduced incidence of sleep disturbances, which include difficulty in falling asleep, multiple awakenings during sleep, disrupted sleep-wake rhythms, and early morning awakenings.

The composition upon oral administration can provide reduced incidence of sundowning.

The composition upon oral administration can provide reduced incidence of gastrointestinal side effects selected from the group of symptoms consisting of nausea, vomiting and diarrhea.

The composition can exhibit an *in vitro* dissolution profile, wherein less than about 20% w/w of donepezil is released in 4 hours in 900ml 0.1N HCl; wherein less than about 20% w/w of donepezil is released in 4 hours in 900ml pH 6.8 phosphate buffer; about20 to 90% w/w of donepezil is released in 4 to 12 hours in 900ml pH 6.8 phosphate buffer and more than about 90% w/w of donepezil is released after 12 hours in 900ml pH 6.8 phosphate buffer.

The single dose AUC₍₁₄₋₂₂₎ of the composition administered in the morning may be 5% to 30%, preferably 5% to 25%, or more preferably 10% to 25%, and most preferably 10% to 20% less than the single dose AUC₍₂₋₁₀₎ of an immediate release composition of donepezil administered in the night, wherein both the compositions have equivalent dose of donepezil.

The steady state AUC₍₁₄₋₂₂₎ of the composition administered in the morning may be 5% to 25%, preferably 5% to 20%, or more preferably 5% to 15%, and most preferably 1% to 10% less than the steady state AUC₍₂₋₁₀₎ of an immediate release composition of donepezil administered in the night, wherein both the compositions have equivalent dose of donepezil.

The single dose AUC₍₇₋₁₀₎ of the composition administered in the morning may be 25% to 85%, preferably 40% to 80%, preferably 45% to 75%, more preferably 50% to 70%, and most preferably 55% to 65% more than the single dose AUC₍₁₉₋₂₂₎ of an immediate release composition of donepezil administered in the night, wherein both the compositions have equivalent dose of donepezil.

The steady state AUC₍₇₋₁₀₎ of the composition administered in the morning may be 10% to 60%, preferably 25% to 55%, preferably 30% to 50%, more preferably 35% to 45% and most preferably 40% to 50% more than the steady state AUC₍₁₉₋₂₂₎ of an immediate release composition of donepezil administered in the night, wherein both the compositions have equivalent dose of donepezil.

### Detailed Description of the Invention

The commercially available compositions of donepezil are at odds with the physiology of the cholinergic system, which is active during the day and quiescent at night. These compositions are so administered that they are active at the night and interfere with NREM sleep and promote REM sleep. This results in sleep disturbances in subjects treated with donepezil.

Further, sundowning syndrome is noticeable in older adults with AD and is believed to be associated with disrupted biological rhythms. Some studies have suggested that increase in acetylcholinesterase is responsible for this phenomenon.

The specification discloses a timed release pharmaceutical composition of donepezil, which provides a plasma donepezil concentration profile that corresponds with the circadian rhythm of the ACh levels in the brain. The release of donepezil from the composition is such that it provides lower levels of donepezil at night and higher levels of donepezil during daytime when compared to the commercially available compositions of donepezil (Aricept® or Aricep®).

The pharmaceutical compositions of donepezil demonstrate similar or greater efficacy and also result in lower incidence of side effects as compared to the commercially available compositions of donepezil.

### Definitions

The term "donepezil" includes various forms of donepezil such as pharmaceutically acceptable salt(s), hydrate(s), solvate(s), polymorph(s), isomer(s), stereoisomer(s), enantiomer(s), racemate(s), ester(s), prodrug(s), derivative(s), analogue(s), metabolite(s) and complex(es) thereof. Donepezil may be present from 0.5 to 90% w/w, preferably 1 to 50% w/w and more preferably 1.5 to 25% w/w of the total weight of the composition.

The term "pharmaceutically acceptable salt" means a salt which is acceptable for administration to a subject, such as a mammal (e.g., salts having acceptable mammalian safety for a given dosage regime). Such salts can be derived from pharmaceutically acceptable inorganic or organic bases and from pharmaceutically acceptable inorganic or organic acids. The preferred salt is donepezil hydrochloride (HC1).

The term "timed release pharmaceutical composition" refers to a composition which is designed to release specific amounts of donepezil in set periods of time such that the resulting plasma donepezil concentration profile in the subject simulates the circadian rhythm of ACh levels in the brain. The release of donepezil is delayed by about 2 to 6 hours after administration followed by release of more than 90% of donepezil 12 hours after administration.

The term "dissolution profile" refers to a plot of the cumulative amount of active ingredient released as a function of time. The dissolution profile is characterized by the test conditions selected. Thus the dissolution profile can be generated at a preselected apparatus type, shaft speed, temperature, volume, and pH of the dissolution media. The dissolution apparatus can be selected from the basket type or the paddle type. The shaft speed can be 100 rotations per minute (RPM) for the basket type apparatus and can be selected from 50 to 75 RPM for the paddle type apparatus. Decreasing or increasing the shaft speed in the range of 25 to 150 RPM can be justified if supported by the dissolution data obtained. The temperature should be 37°C. The volume of the apparatus can be selected from 500mL to 1000mL, most preferably 900mL. The dissolution media used are dilute HCl (0.1N), buffers in the pH range 1.2 - 6.8, simulated gastric or intestinal fluid and water.

"Circadian rhythm" is the regular 24 hours rest/wake pattern seen in normal individuals. ACh levels in the brain also follow a circadian rhythm wherein there are increased ACh levels during waking and motor activity and decreased levels, in general, during sleep.

The sleep-wake cycle or rhythms comprises three main phases: wakefulness, non-rapid eye movement (NREM) sleep and rapid eye movement (REM) sleep. On the basis of the depth of sleep, NREM sleep is subdivided into stages. In healthy young adults, sleep usually begins at the lightest stage, NREM stage 1, and progresses through stage 2 and into the deepest sleep states, stage 3 and stage 4, also known as delta or slow-wave sleep. The sleeper then progresses back to lighter sleep and then into REM sleep. In healthy individuals, a typical 8-hour period of sleep contains four or five cycles of these alternating sleep phases, with occasional brief awakenings. REM sleep accounts for 20-25% of total sleep time in most human adults.

Sleep stages are identified by using an electroencephalogram to detect the electrical activity in the cerebral cortex, an electrooculogram to monitor eye movements, and an electromyogram to measure muscle activity. The combination of these three recordings is referred to as polysomnography.

Sleep quality is defined by the nighttime sleep characteristics revealed by self-report or polysomnography. It usually includes sleep onset latency, waking frequency, durations of awakening after sleep onset, amount of nighttime sleep, and sleep efficiency.

"Sleep onset latency time" refers to the length of time that it takes to accomplish the transition from full wakefulness to sleep, normally to the lightest of the non-REM sleep stages.

"REM sleep cycles" refers to the number of times the sleeper enters into the REM phase of sleep cycle during a normal nighttime sleep.

"REM phase duration" refers to amount of time the sleeper remains in the REM phase during a normal nighttime sleep.

"Sleep efficiency" refers to the ratio of the time a sleeper is asleep to the time the sleeper spends in the bed.

"Parasomnia" refers to a category of sleep disorders that involve abnormal and unnatural movements, behaviors, emotions, perceptions, and dreams that occur while falling asleep, sleeping, between sleep stages, or during arousal from sleep. Most parasomnias are dissociated sleep states which are partial arousals during the transitions between wakefulness and NREM sleep, or wakefulness and REM sleep.

"REMS parasomnia" refers to a category of sleep disorders that involve abnormal and unnatural movements, behaviors, emotions, perceptions, and dreams that occur during the REM phase of sleep.

"REM sleep behavior disorder" is a sleep disorder (parasomnia) that involves abnormal behavior during the REM sleep. In a person with REM sleep behavior disorder (RBD), the paralysis that normally occurs during REM sleep is incomplete or absent, allowing the person to "act out" his or her dreams. RBD is characterized by the acting out of dreams that are vivid, intense, and violent. Dream-enacting behaviors include talking, yelling, punching, kicking, sitting, jumping from bed, arm flailing, and grabbing.

"Sundowning" is the appearance or exacerbation of behavioral disturbances associated with the afternoon and/or evening hours in subjects suffering from Alzheimer's. Individual behavioral components of the condition which is frequently termed sundowning in subjects suffering from Alzheimer's may include anxiety, agitation, locomotor activity, delirium, carphologic behavior (restlessness), escape behaviors, expression of feelings, talking and other verbalizations, appearance of searching, combativeness, purposeless movement, wandering, loud/prolonged incoherent vocalization, hallucinations, confusion, disorientation, restraint removal, searching, tapping or banging etc. These behaviors usually start at around 4 PM and last as late as 11 PM.

The timed release pharmaceutical composition of donepezil can be characterized by its pharmacokinetic (pK) parameters which describes the in vivo characteristics of donepezil over time. These pK parameters include Cₘₐₓ, Tₘₐₓ, AUC, AUC_{(X-Y)}.

Cₘₐₓ is the "maximum plasma concentration" which is defined as the concentration of donepezil in the plasma at the point of maximum concentration.

"Tₘₐₓ" refers to the time at which the concentration of donepezil in the plasma is the highest.

The term "AUC" as used herein means "area under the curve" in a plot of concentration of drug in plasma versus time from ingestion. AUC is usually given for the time interval zero to any time 't' post drug administration or extrapolated to infinity. AUC zero to infinity is estimated based on mathematical approaches using limited number of concentration measurements.

"AUC_{(X-Y)}" is the "area under the curve" in a plot of concentration of drug in plasma versus time, measured from time point X hours after administration of the composition to time point Y hours after administration of the composition.

The pK parameters can be measured after administration of a single dose of the composition or after administration of multiple doses of the composition such that steady state of donepezil is attained.

"Single dose AUC_{(X-Y)}" relates to the AUC_{(X-Y)} which is measured after administration of a single dose of the composition. "Steady state AUC_{(X-Y)}" relates to the AUC_{(X-Y)} which is measured after administration of the composition on a daily basis for at least 14 days.

"Immediate release" refers to a conventional or non-modified release composition, in which greater than or equal to about 75% of the active agent is released within 45 minutes of administration, preferably within 30 minutes of administration.

"Rescue anti-acid drugs" refer to use of anti-acid drugs such as proton pump inhibitors (PPIs), histamine 2 receptor antagonists (H2RAs) or antacids to manage GI side-effects which become persistent or which recur or which get worse on donepezil therapy.

"Titration period" refers to a time period required for gradually adjusting the dose of a medication to reach the optimum therapeutic dose with acceptable tolerability.

"Commercially available compositions of donepezil" comprises of immediate release and sustained release compositions of donepezil available under the tradename ARICEPT®.

The "immediate release composition of donepezil" include "ARICFPT® OR ARICEP®" film coated tablets, comprising 5 & 10 mg of donepezil hydrochloride, and inactive ingredients containing lactose monohydrate, cornstarch, microcrystalline cellulose, hydroxypropyl cellulose, and magnesium stearate. The film coating contains talc, polyethylene glycol, hydroxypropyl methylcellulose, and titanium dioxide. The 10 mg tablet further includes yellow iron oxide as a coloring agent. The immediate release composition of donepezil attains Tₘₐₓ 2 to 5 hours after single dose administration.

The "sustained release composition of donepezil" includes "ARICEPT®" film coated tablets comprising 23mg of donepezil hydrochloride and inactive ingredients containing ethylcellulose, hydroxypropyl cellulose, lactose monohydrate, magnesium stearate and methacrylic acid copolymer, Type C. The film coating includes ferric oxide, hypromellose 2910, polyethylene glycol 8000, talc and titanium dioxide. The sustained release composition of donepezil attains Tₘₐₓ 8 hours after single dose administration.

The composition can exhibit an *in vitro* dissolution profile, wherein less than about 20% w/w of donepezil is released in 4 hours in 900ml 0.1N HC1; wherein less than about 20% w/w of donepezil is released in 4 hours in 900ml pH 6.8 phosphate buffer about 20 to 90% w/w of donepezil is released in 4 to 12 hours in 900ml pH 6.8 phosphate buffer and more than about 90% w/w of donepezil is released after 12 hours in 900ml pH 6.8 phosphate buffer

The single dose AUC₍₁₄₋₂₂₎ of the composition administered in the morning may be 5% to 30%, preferably 5% to 25%, or more preferably 10% to 25%, and most preferably 10% to 20% less than the single dose AUC₍₂₋₁₀₎ of an immediate release composition of donepezil administered in the night, wherein both the compositions have equivalent dose of donepezil.

The steady state AUC₍₁₄₋₂₂₎ of the composition administered in the morning may be 5% to 25%, preferably 5% to 20%, or more preferably 5% to 15%, and most preferably 1% to 10% less than the steady state AUC₍₂₋₁₀₎ of an immediate release composition of donepezil administered in the night, wherein both the compositions have equivalent dose of donepezil.

The single dose AUC₍₇₋₁₀₎ of the composition administered in the morning may be 25% to 85%, preferably 40% to 80%, preferably 45% to 75%, more preferably 50% to 70%, and most preferably 55% to 65% more than the single dose AUC₍₁₉₋₂₂₎ of an immediate release composition of donepezil administered in the night, wherein both the compositions have equivalent dose of donepezil.

The steady state AUC₍₇₋₁₀₎ of the composition administered in the morning may be 10% to 60%, preferably 25% to 55%, preferably 30% to 50%, more preferably 35% to 45% and most preferably 40% to 50% more than the steady state AUC₍₁₉₋₂₂₎ of an immediate release composition of donepezil administered in the night, wherein both the compositions have equivalent dose of donepezil.

These pK parameters of the composition are compared to those of an immediate release composition of donepezil, preferably between compositions containing equivalent dose of donepezil.

The pK parameters of the composition are also compared to the commercially available sustained release composition comprising 23mg of donepezil hydrochloride (Aricept®).

The pK parameters of the composition are also compared to commercially available compositions of donepezil wherein the compositions have different doses of donepezil. In such cases, the dose corrected partial AUC is measured. The dose corrected partial AUC is the partial AUC divided by the number of milligrams of donepezil in the formulation. The timed release pharmaceutical composition of donepezil provides a plasma donepezil concentration profile that corresponds with the circadian rhythm of the ACh levels in the brain.

The composition can reduce incidence of sleep disturbances, which includes difficulty in falling asleep, multiple awakenings during sleep, disrupted sleep-wake rhythms, and early morning awakenings.

The composition upon oral administration can provide reduced incidence of insomnia and nightmares and reduced sleep onset latency time. It can reduce REM sleep cycles, REM phase duration, waking frequencies and the durations of awakening after sleep onset; prolong the nighttime sleep duration, improve sleep efficiency, provide reduced incidence of REMS parasomnia and provide reduced incidence of behavioral and motor disturbances in the REM sleep behavior disorder.

The composition upon oral administration can provide reduced incidence of sundowning.

The composition upon oral administration can provide reduced incidence of gastrointestinal side effects selected from the group consisting of nausea, vomiting and diarrhea. The composition can reduce the need for use of rescue anti-acid drugs in subjects being treated with donepezil.

The composition can shorten the titration period required for donepezil administration. Preferably, the composition shortens the titration period required for donepezil administration by two to four weeks.

The composition can be used to treat a subject suffering from a disease or a condition characterized by symptoms of dementia and/or cognitive impairments. Such disease or condition can be selected from the group consisting of Alzheimer's disease, mild cognitive impairment (MCI), senile dementia, vascular dementia, dementia of Parkinson's disease, attention deficit disorder, attention deficit hyperactivity disorder (ADHD), dementia associated with Lewy bodies, AIDS dementia complex (ADC), Pick's disease, dementia associated with Down's syndrome, amyotrophic lateral sclerosis, Huntington's disease, cognitive deficits associated with traumatic brain injury (TBI), cognitive and sensorimotor gating deficits associated with schizophrenia, cognitive deficits associated with bipolar disorder, cognitive impairments associated with depression, delirium, schizoaffective disorder, aphasia, autism, schizophreniform disorder, obstructive sleep apnea, sleep deprivation, cerebrovascular accident, relapsing remitting multiple sclerosis, ischemic stroke, anxiety disorder.

Preferably, the composition is used to treat a subject suffering from mild, moderate or severe Alzheimer' s disease.

The timed release pharmaceutical composition of donepezil comprises donepezil or a pharmaceutically acceptable salt thereof in an amount selected from 3mg to 50mg. For example, the composition may comprise donepezil in an amount selected from 5mg to 25mg, from 10mg to 25mg, from 7mg to 18mg. The pharmaceutical composition may comprise 3mg, 4mg, 5mg, 6mg, 6.5mg, 7mg, 7.5mg, 8mg, 8.5mg, 9mg, 9.5mg, 10mg, 11mg, 12mg, 13mg, 14mg, 15mg, 16mg, 17mg, 17.5mg, 18mg, 18.5mg, 19mg, 20mg or 23mg of donepezil.

The timed release pharmaceutical composition can provide dose reduction from about 5-50%, preferably 10-45%, preferably, 15-40% of the standard dose (5mg, 10mg and 23mg) without reducing the efficacy.

The dosage regimen for treating and preventing the diseases described herein with donepezil can be selected in accordance with a variety of factors, including the age, weight, sex, and medical condition of the subject, the route of administration, pharmacological considerations such as the activity, efficacy, pharmacokinetic and toxicology profiles of the active ingredient, and whether a drug delivery system is used. The doses can be administered in one to four portions over the course of a day, preferably once a day.

The timed release pharmaceutical composition of donepezil maybe administered in two portions over the course of a day, wherein the amount of donepezil present in the two portions is same or different. Preferably, the amount of donepezil present in the two portions is different and wherein the portion administered in the night has a greater amount of donepezil than the portion administered in the morning. In a preferred embodiment, the pharmaceutical composition of donepezil comprising 16mg, 17mg, 17.5mg, 18mg or 18.5mg of donepezil is administered in the night and pharmaceutical composition of donepezil comprising 6.5mg, 7mg, 7.5mg, 8mg, 8.5mg or 9mg of donepezil is administered in the morning.

The term active ingredient, active agent and drug herein can be interchangeably used.

The composition can be administered to the subject in the morning or in the evening or night. Preferably, the composition is administered in the morning. Preferably the composition is administered between 6 a.m. and 10a.m. and more preferably, the composition is administered at 8 a.m.

The composition is particularly suitable for oral administration. The composition include but is not limited to tablets (single layered tablets, multilayered tablets, mini tablets, bioadhesive tablets, caplets, matrix tablets, tablet within a tablet, mucoadhesive tablets, gastroretentive tablets), pellets, beads, granules, capsules, microcapsules, tablets in capsules and microspheres, matrix formulations, microencapsulation and powder/pellets/granules for suspension. Powders, pellets, and granules may be coated with a suitable polymer or a conventional coating material to achieve, for example, greater stability in the gastrointestinal tract, or to achieve the desired rate of release. Moreover, capsules containing a powder, pellets, or granules may be further coated. It may also include kits. Tablets are most preferably used.

The composition comprises donepezil and one or more release controlling agent(s) to produce a timed release profile. The release controlling agent(s) is/are selected from hydrophilic release controlling agents, hydrophobic release controlling agents, natural release controlling agents and synthetic release controlling agents. The release controlling agent maybe selected from hydrophilic or hydrophobic agents, which can be polymeric or non- polymeric and which are capable of modulating the rate of release of the active ingredient(s), which can be pH dependent or pH independent. The release controlling agent(s) may be natural, semi-synthetic and synthetic agents or mixtures thereof. The amount of release controlling agent is 10 to 50% w/w of the total composition.

The hydrophilic release controlling agents maybe selected from hydroxyethylcellulose, hydroxypropyl cellulose, hydroxypropyl methylcellulose, sodium carboxymethyl cellulose, sodium alginate, carbomer (Carbopol(TM)), xanthan gum, guar gum, locust bean gum, poly vinyl acetate, polyvinyl alcohol. Preferably the release-controlling agent is hydroxypropylmethylcellulose.

The hydrophobic release controlling agents maybe selected from hydrogenated vegetable oil, purified grades of beeswax; fatty acids; long chain fatty alcohols, such as cetyl alcohol, myristyl alcohol, and stearyl alcohol; glycerides such as glyceryl esters of fatty acids like glyceryl monostearate, glyceryl distearate, glyceryl esters of hydrogenated castor oil and the like; oils such as mineral oil and the like, or acetylated glycerides; ethyl cellulose, stearic acid, paraffin, carnauba wax, talc; and the stearate salts such as calcium, magnesium, zinc and other materials known to one of ordinary skill in the art.

Natural release controlling agents maybe selected from proteins (e.g., hydrophilic proteins), such as pectin, zein, modified zein, casein, gelatin, gluten, serum albumin, or collagen, chitosan, oligosaccharides and polysaccharides such as cellulose, dextrans, tamarind seed polysaccharide, gellan, carrageenan, xanthan gum, gum Arabic, guar gum, locust bean gum; hyaluronic acid, polyhyaluronic acid, alginic acid, sodium alginate or combinations thereof

Synthetic release controlling agents maybe selected from polyamides, polycarbonates, polyalkylenes, polyalkylene glycols such as poly(ethylene glycol), polyalkylene oxides, polyethylene oxide, polyalkylene terephthalates, polyvinyl alcohols (PVA), polyvinylphenol, polyvinyl ethers, polyvinyl esters, polyvinyl halides, polyvinylpyrrolidone(PVP), polyglycolides, polysiloxanes, polyurethanes, polystyrene, polylactides, poly (butyric acid), poly (valeric acid), poly(lactide-co-glycolide), poly (ethyleneterephthalate), poly (lactide-co-caprolactone), polyanhydrides (e.g., poly (adipic anhydride), polyorthoesters, poly(fumaric acid), poly(maleic acid), polyvinyl acetate, polystyrene; polymers of acrylic and methacrylic esters(available under the trade name Eudragit® like Eudragit® RSPO, Eudragit® RLPO, Eudragit® L100-55); carbomer, carbopol®; celluloses and cellulose derivatives such as methyl cellulose(MC), ethyl cellulose(EC), hydroxypropyl cellulose(HPC), hydroxypropylmethyl cellulose (HPMC), hydroxybutylmethyl cellulose, hydroxyl ethyl cellulose (HEC) sodium carboxymethyl cellulose(Sod.CMC), cellulose acetate(CA), cellulose propionate, cellulose acetate butyrate, cellulose acetate phthalate(CAP), carboxymethyl cellulose, cellulose triacetate, cellulose sulfate sodium salt and blends and copolymers thereof or mixtures thereof.

The composition may further comprise pharmaceutically acceptable excipients. Examples of pharmaceutically acceptable excipients may include diluents, binders, disintegrants, lubricants, glidants, and coloring agents. The amount of additive employed depends upon various factors such asamount of active agent to be used.

Diluents maybe selected from alumina, starch, kaolin, polacrilin potassium, powdered cellulose, microcrystalline cellulose, sugars such lactose, glucose, fructose, sucrose, mannose, dextrose, galactose, dextrates, dextrin; sugar alcohols such as mannitol, sorbitol, xylitol, lactitol, starch, calcium carbonate, calcium phosphate dibasic or tribasic, calcium sulphate or combinations thereof.

Binders maybe selected from starches such as potato starch, wheat starch, corn starch; microcrystalline cellulose such as products known under the registered trademarks Avicel, Filtrak, Heweten or Pharmacel; celluloses such as HPC, HEC, HPMC, EC, sodium carboxy methyl cellulose; natural gums like acacia, alginic acid, guar gum; liquid glucose, dextrin, povidone, syrup, polyethylene oxide, PVP, poly-N-vinyl amide, polyethylene glycol, gelatin, poly propylene glycol, tragacanth, combinations thereof and other materials known to one of ordinary skill in the art and mixtures thereof.

Disintegrants may be selected from low-substituted hydroxypropyl cellulose, e.g. L-HPC; cross-linked polyvinyl pyrrolidone (PVP-XL), e.g. Kollidon® CL and Polyplasdone® XL; cross-linked sodium carboxymethylcellulose, e.g. Ac-di-sol®, Primellose®; sodium starch glycolate, e.g. Primojel®; sodium carboxymethylcellulose; sodium carboxymethyl starch, e.g. Explotab®; ionexchange resins, e.g. Dowex® or Amberlite® ; microcrystalline cellulose, e.g. Avicel® ; starches and pregelatinized starch, e.g. Starch 1500® , Sepistab ST200® ; formalin casein, e.g. Plas-Vita® and combinations thereof.

Lubricants may be selected from those conventionally known in the art such as magnesium, aluminum or calcium or zinc stearate, silicon oxide, polyethylene glycol, glyceryl behenate, mineral oil, talc, sodium stearyl fumarate, stearic acid, vegetable oil such as hydrogenated vegetable oiland combinations thereof.

Glidants may be selected from silicon dioxide; magnesium trisilicate, talc and tribasic calcium phosphate, calcium silicate, magnesium silicate, colloidal silicon dioxide, silicon hydrogel.

Coloring agents maybe selected from ferric oxide; pigments such as titanium dioxide. Colorants can also include natural food colors and dyes suitable for food, drug and cosmetic applications.

The amount of each type of excipient employed, e.g. glidant, binder, disintegrant, filler or diluent and lubricant may vary. Thus for example, the amount of glidant may vary within a range of 0.1 to 10% by weight, in particular 0.1 to 5% by weight, e.g. 0.1 to 0.5% by weight; the amount of binder may vary within a range of from about 0.5to 45% by weight, e.g. 20 to 30% by weight; the amount of disintegrant may vary within a range of from 0.5 to 5% by weight, e.g. 1% by weight; the amount of filler or diluent may vary within a range of from 10 to 60% by weight; whereas the amount of lubricant may vary within a range of from 0.1 to 5.0% by weight.

One excipient can perform more than one function.

The composition can be prepared by various methods known in the art such as by dry granulation, wet granulation, melt granulation, direct compression, double compression, extrusion spheronization, layering and the like. The pharmaceutical composition can be in any shape and size.In an embodiment, the tablets are round with initial diameter of 13.3mm.

In an embodiment, the process of making the composition comprises as described below:
i) blending donepezil and pharmaceutically acceptable additives,
ii) subjecting the blend to slugging/compaction to form a coprimate,
iii) converting the coprimate to granules,
iv) compressing the granules to form the solid oral dosage form, and
v) optionallycoating the solid oral dosage form.

Compaction of the blend into coprimate may be carried out using a slugging technique or roller compaction. The milling of the granules may be carried out according to conventional milling methods.

The process of wet granulation includes aqueous or non-aqueous granulation. The wet granulation process comprises the admixing of the active ingredient with diluent(s) and/or rate controlling polymer, and granulation of the blend with the binder mass to form the wet mass followed by drying and sizing. The binder may optionally be admixed with the dry blend and granulation performed with aqueous or non-aqueous solvent. The solvent for the non-aqueous granulation is selected from ethanol, isopropyl alcohol and dichloromethane or mixtures thereof.

The composition comprises a functional coating. The composition may further be coated with a non-functional coating. The coating may comprise about 1% to about 40%, about 3% to about 30%, about 5% to about 25%, about 6% to about 10%, about 1% to about 10%, about 1 % to about 5%, about 2% to about 10%, about 2% to about 8% of the total composition. The coating materials for use in the embodiments disclosed herein includes, but is not limited to, natural, semisynthetic and synthetic agents or combinations thereof.

The inclusion of an effective amount of a plasticizer in the coating may improve the physical properties of the coating. Generally, the amount of plasticizer included in a coating solution is based on the concentration of the polymer, e.g., most often from about 1 to about 50 percent by weight of the polymer. Concentrations of the plasticizer, however, can be determined by routine experimentation. Examples of plasticizers include plasticizers such as dibutyl sebacate, phthalate esters such as diethyl phthalate, dibutyl phthalate, triacetin, acetylated monoglycerides, castor oil, citric acid esters such as triethyl citrate, tributyl citrate, 1,2-propylene glycol, polyethylene glycols, propylene glycol, acetin or combinations thereof.

Suitable methods can be used to apply the coating such assimple or complex coacervation, interfacial polymerization, liquid drying, thermal and ionic gelation, spray drying, spray chilling, fluidized bed coating, pan coating, electrostatic deposition, compression coating, hot melt (extrusion) coating. Such methods are well known to those skilled in the art.

The solvent may be selected alcoholic or hydroalcoholic. Alcoholic solvents may be selected from methanol, ethanol, isopropyl alcohol, halogenated hydrocarbons such as dichloromethane (methylene chloride), hydrocarbons such as cyclohexane, and acetone and combinations thereof.

The composition is a coated composition, which comprises: a) a core comprising donepezil and one or more release controlling agent(s), and b) a functional coating comprising one or more release controlling agent(s).

The composition may be a multi-layered composition, wherein the composition is core-shell type in which a gastroretentive component and delayed controlled release component make a bilayer tablet as a core or a tablet-in-tablet as a core in which delayed controlled release component as inner core and gastroretentive component as outer core. The controlled release layer/component comprises donepezil and one or more release controlling agent(s). The gastroretentive layer/component maybe a bioadhesive layer and/or a swellable layer.

Gastro retention of the pharmaceutical composition maybe achieved by using swelling and expanding systems which are retained by virtue of size of the pharmaceutical composition that is more than the size of the pyloric sphincter e.g. Plug-Type systems. Preferably, the initial diameter of such compositions should be 13.3mm. In this type of gastroretentive technique, release controlling agents imbibe water and swell enough to be retained in the upper part of the GIT.

The composition can be a bioadhesive or mucoadhesive composition, wherein the composition can be retained in any part of the gastrointestinal tract (GIT) for increasing the GIT residence time, to increase the exposure of the composition to the GIT thus facilitating extended period of absorption of the active ingredient. Bioadhesive and mucoadhesive can be used interchangeably.

### Animal studies

The effect of the composition in reducing the incidence of sundowning effect can be demonstrated using animal studies with aged mice or amyloid precursor protein (APP) transgenic mice or other appropriate animal species. The following endpoints maybe measured at different time points of light and dark cycle:
a) Behavioral parameters indicating sundowning:
   - Anxiety-like behavior
   - Locomotor activity
b) AChE levels measurement
c) Body temprature measurement

### Clinical studies

### I. Single-dose cross over PK study AUC results (Donepezil MR morning-time Versus Donepezil IR Night-Time)

The results of Single-dose cross over PK study AUC results (Donepezil MR morning-time Versus Donepezil IR Night-Time) are disclosed in Table 1.

**Table 1: AUC differences: Donepezil MR morning-time Versus Donepezil IR Night-Time**

| | Donepezil IR - night-time | Donepezil MR-Morning-time |
|---|---|---|
| AUC2-10 | 71.47 | |
| AUC14-22 | | 49.95 |
| % Difference | -29.466 | |

| | | |
|---|---|---|
| * (minus (-) sign indicates lower AUC; (+) sign indicates higher value) than Donepezil IR | | |

### II. Polysomnography studies:

Polysomnography studies can be conducted to assess the reduction in sleep disturbances caused by the time release pharmaceutical composition of donepezil as compared to the immediate release compositions of donepezil. The studies can be single or multiple dose, two or three arm and parallel or cross-over studies conducted in healthy volunteers or AD patients. The following parameters can be assessed:
a) EEG wave pattern for REM and NREM sleep
b) Total duration of REM sleep between the groups
c) Frequency of REM sleep between the groups
d) Time taken to fall asleep (sleep latency) after dose
e) Total number of awakenings after initially falling asleep
f) Total duration remained awake
g) Total sleep duration
h) Sleep efficiency between groups

Clinical studies can be conducted to evaluate the reduction in gastrointestinal side effects caused by the composition as compared to the immediate release composition of donepezil. The studies can be single dose, two or three period cross-over studies conducted in healthy volunteers. The following parameters can be assessed:
a) Incidence of any GI side-effects such as nausea, vomiting, dyspepsia etc between the groups
b) Comparing incidences of GI side-effects across all dose levels in between groups
c) Measurement or rating of nausea, vomiting, dyspepsia and other GI symptoms using visual analog sales or questionnaires
d) Proportion of subjects who required use of rescue anti-acid drugs and comparison between the groups

The foregoing examples are illustrative embodiments and are merely exemplary.

### Example 1:

| **Ingredients** | **Quantity in % w/w** |
|---|---|
| Donepezil HCl | 2.0 |
| Hydroxy Propyl Methyl Cellulose | 26.7 |
| Lactose | 40.1 |
| Sodium Starch Glycolate | 7.3 |
| Hydroxy Propyl Cellulose | 4.0 |
| Microcrystalline Cellulose | 11.5 |
| Colloidal Silicon Dioxide | 0.9 |
| Magnesium Stearate | 0.9 |
| Purified water | qs |

| **Coating** | |
|---|---|
| Ethyl Cellulose | 2.65 |
| Hydroxy Propyl Methyl Cellulose | 2.65 |
| Triethyl Citrate | 1.3 |
| Isopropyl Alcohol | qs |
| Dichloromethane | qs |
| **Total Coated Tablet Weight** | 100 |

### Brief Manufacturing procedure:

1. Donepezil HC1, sodium starch glycolate, hydroxylpropylmethylcellulose, lactose and hydroxy propyl cellulose are weighed, sifted and mixed together.
2. The mixture of step 1 is granulated using purified water with suitable granulation parameters and the granules are then dried.
3. The dried granules are sifted through a suitable sieve.
4. The remaining quantities of hydroxylpropylmethylcellulose, lactose and microcrystalline cellulose are sifted through a suitable sieve and mixed with the granules of step 3.
5. Colloidal silicon dioxide and magnesium stearate are sifted through a suitable sieve and mixed with the blend of step 4.
6. The lubricated blend of step 5 is compressed using a round shape punch with suitable physical parameters.
7. Ethyl cellulose, hydroxypropylmethyl cellulose and triethyl citrate are dissolved in a mixture of isopropyl alcohol and dichloromethane with continuous stirring.
8. The compressed tablets of step 6 are coated using coating solution of step 7 with suitable coating parameters.
9. Dissolution was carried out in USP-II paddle apparatus in 0.1N HCl and 6.8 pH Phosphate buffer (PB) media. (Table 2)

**Table 2: Dissolution data of donepezil formulation prepared according to Example 1.**

| Time (hr) | Dissolution in0.1N HCl (% Donepezil) | Dissolution in 6.8 pH P.B (% Donepezil) |
|---|---|---|
| 0 | 0 | 0 |
| 3 | 0 | 0 |
| 4 | 1 | 3 |
| 5 | 6 | 19 |
| 6 | 22 | 40 |
| 8 | 67 | 73 |
| 10 | 92 | 84 |
| 12 | 101 | 86 |
| 14 | 103 | 89 |

### Example 2:

| **Ingredients** | **Quantity in % w/w** |
|---|---|
| Donepezil HCl | 2.00 |
| Hydroxy Propyl Methyl Cellulose | 26.70 |
| Lactose | 40.10 |
| Sodium Starch Glycolate | 7.30 |
| Hydroxy Propyl Cellulose | 4.00 |
| Microcrystalline Cellulose | 11.50 |
| Colloidal Silicon Dioxide | 0.90 |
| Magnesium Stearate | 0.90 |
| Purified water | qs |

| **Coating** | |
|---|---|
| Eudragit RSPO | 2.65 |
| Eudragit L100-55 | 2.65 |
| Triethyl Citrate | 1.30 |
| Isopropyl Alcohol | qs |
| Acetone | qs |
| **Total Coated Tablet Weight** | 100 |

Brief Manufacturing procedure:
1. Donepezil HC1, sodium starch glycolate, hydroxypropylmethylcellulose, lactose and hydroxy propyl cellulose are weighed, sifted and mixed together.
2. The mixture of step 1 is granulated using purified water with suitable granulation parameters and the granules are then dried.
3. The dried granules are sifted through a suitable sieve.
4. The remaining quantities of hydroxylpropylmethylcellulose, lactose and microcrystalline cellulose are sifted through a suitable sieve and mixed with the granules of step 3.
5. Colloidal silicon dioxide and magnesium stearate are sifted through a suitable sieve and mixed with the blend of step 4.
6. The lubricated blend of step 5 is compressed using a round shape punch with suitable physical parameters.
7. Eudragit RSPO, Eudragit L100-55, hydroxypropylmethyl cellulose and triethyl citrate are dissolved in a mixture of isopropyl alcohol and acetone with continuous stirring.
8. The compressed tablets of step 6 are coated using coating solution of step 7 with suitable coating parameters.

### Example 3:

| **Ingredients** | **Quantity in % w/w** |
|---|---|
| Hardened Sugar Spheres | 50.00 |
| Donepezil HCl | 8.33 |
| Hydroxy Propyl Methyl Cellulose | 4.50 |
| Hydroxy Propyl Cellulose | 8.01 |
| Talc | 1.94 |
| Triethyl Citrate | 0.28 |
| Isopropyl Alcohol | qs |
| Methylene Chloride | qs |
| Purified water | qs |

| **Coating** | |
|---|---|
| Eudragit RLPO | 2.78 |
| Eudragit RSPO | 19.44 |
| Dibutyl Sebacate | 4.72 |
| Isopropyl Alcohol | qs |
| Acetone | qs |
| **Total Weight of Pellets** | 100.00 |
| Empty Hard Gelatin Capsules | |

Brief Manufacturing procedure:
1. Sugar Spheres Hardening
   1.1 The sugar spheres are sifted using vibratory sifter.
   1.2 Triethyl citrate and hydroxypropylmethylcellulose are dissolved in a mixture of Isopropyl alcohol and methylene chloride under continuous stirring.
   1.3 The sugar spheres are loaded in the fluidized bed processor and the fluidization is started.
   1.4 The entire solution of step 1.2 is sprayed onto the sugar spheres at a suitable temperature.
   1.5 The hardened sugar spheres of step 1.4 are dried in the fluidized bed processor at suitable temperature.
   1.6 The dried hardened sugar spheres of step 1.5 are sifted through suitable sieves and the fines are discarded.
2. Drug Loading
   2.1 Hydroxy propyl cellulose is added to purified water to obtain a clear solution.
   2.2 Donepezil hydrochloride is added to the solution of step 2.1 to obtain a clear solution.
   2.3 Talc is added to the solution of step 2.2 under continuous stirring and the dispersion is filtered through a suitable sieve.
   2.4 The dried hardened sugar spheres of step 1.6 are loaded in the fluidized bed processor and the fluidization is started.
   2.5 The entire dispersion of step 2.3 is sprayed onto the hardened sugar spheres at a suitable temperature.
   2.6 The drug loaded pellets of step 2.5 are sifted through suitable sieves and the agglomerates and fines are discarded.
3. Coating step
   3.1 Eudragit and dibutyl sebacate are dissolved in a mixture of Isopropyl alcohol and Acetone with continuous stirring.
   3.2 The drug-loaded pellets of step 2.6 are coated using the solution of step 3.1 with suitable coating parameters.
   3.3 The coated pellets of step 3.2 are filled in hard gelatin capsule shells with required fill weight.

### Example 4:

| **Ingredients** | **Quantity in % w/w** |
|---|---|
| Donepezil HCl | 3.6 |
| Eudragit RLPO | 1.2 |
| Eudragit L100-55 | 18.08 |
| Eudragit RSPO | 8.44 |
| Ethyl cellulose | 8.48 |
| Lactose | 27.72 |
| Hydroxy Propyl Cellulose | 4.82 |
| Microcrystalline Cellulose | 17.60 |
| Colloidal Silicon Dioxide | 0.96 |
| Magnesium Stearate | 0.72 |
| Purified water | qs |

| **Coating** | |
|---|---|
| Eudragit L100-55 | 6.02 |
| Triethyl citrate | 2.36 |
| Isopropyl Alcohol | qs |
| Acetone | qs |
| **Total Coated Tablet Weight** | 100 |

### Brief Manufacturing procedure:

1. Donepezil HC1, Eudragit RLPO, Eudragit L100-55, Eudragit RSPO, lactose and hydroxy propyl cellulose are weighed, sifted and mixed together.
2. The mixture of step 1 is granulated using purified water with suitable granulation parameters and the granules are then dried.
3. The dried granules are sifted through a suitable sieve.
4. Microcrystalline cellulose, colloidal silicon dioxide and magnesium stearate are sifted through a suitable sieve and mixed with the blend of step 3.
5. The lubricated blend of step 4 is compressed using a round shape punch with suitable physical parameters.
6. Ethyl cellulose, hydroxypropylmethyl cellulose and dibutyl sebacate are dissolved in a mixture of isopropyl alcohol and dichloromethane with continuous stirring.
7. The compressed tablets of step 5 are coated using coating solution of step 6 with suitable coating parameters.

### Example 5

| **Ingredients** | **Quantity in % w/w** |
|---|---|
| **Active Layer** | |
| Donepezil HCl | 4.76 |
| Polyvinyl pyrrolidone | 1.59 |
| Mannitol | 15.90 |
| Polyethylene Oxide | 14.29 |
| Magnesium stearate | 0.95 |
| Isopropyl Alcohol | qs |

| **Push-pull Layer** | |
|---|---|
| Polyethylene oxide | 7.94 |
| Potassium Chloride | 15.88 |
| Hydroxy propyl methyl cellulose | 14.29 |
| Polyvinyl pyrrolidone | 3.18 |
| Magnesium Stearate | 0.64 |

| **Semi Permeable coating** | |
|---|---|
| Cellulose acetate | 7.94 |
| Triethyl Citrate | 1.59 |
| Acetone | qs |

| **Functional Coating** | |
|---|---|
| Eudragit L100-55 | 7.94 |
| Triethyl Citrate | 3.11 |
| Isopropyl Alcohol | qs |
| Acetone | qs |
| **Total Coated Tablet Weight** | 100 |

Brief Manufacturing Procedure
1. Donepezil HC1, mannitol and polyethylene oxide are sifted and mixed together.
2. The mixture of step 1 is granulated with polyvinyl pyrrolidone solution in isopropyl alcohol.
3. The granules of step 2 are dried and sifted through a suitable sieve.
4. The dried granules of step 3 are lubricated using magnesium stearate.
5. Polyethylene oxide, potassium chloride and hydroxy propyl methyl cellulose are sifted and mixed together and lubricated using magnesium stearate.
6. The granules of step 4 and mixture of step 5 are compressed as bilayered tablets using a round shape punch with suitable physical parameters.
7. Cellulose acetate and triethyl citrate are dissolved in acetone with stirring.
8. The compressed tablets of step 6 are coated using coating solution of step 7 with suitable coating parameters.
9. The coated tablets of step 8 are drilled with laser drilling technology on one or both sides.
10. Eudragit L100-55 and triethyl citrate are dissolved in a mixture of isopropyl alcohol and acetone with continuous stirring.
11. The coated tablets of step 9 are coated using coating solution of step 10 with suitable coating parameters.

## Claims

1. A timed release pharmaceutical composition comprising donepezil or a pharmaceutically acceptable salt thereof, wherein the composition is a coated composition, which comprises:
a) a core comprising donepezil in an amount from 3 mg to 50 mg, and one or more release controlling agent(s), and
b) a functional coating comprising one or more release controlling agent(s);
wherein the release controlling agent(s) is/are selected from hydrophilic release controlling agents, hydrophobic release controlling agents, natural release controlling agents and synthetic release controlling agents;
the amount of release controlling agent in the composition is 10% w/w to 50% w/w by total weight of the composition; and
the composition exhibits the following in vitro dissolution profile when tested in a Paddle dissolution apparatus at 50 rpm in 900 ml pH 6.8 buffer at 37°C less than 20% w/w of donepezil is released in 3-4 hrs, and more than 85% w/w of donepezil is released after 12 hrs.

2. The timed release pharmaceutical composition according to claim 1 wherein the hydrophilic release controlling agent(s) is selected from hydroxyethylcellulose, hydroxypropyl cellulose, hydroxypropyl methylcellulose, sodium carboxymethyl cellulose, sodium alginate, carbomer, xanthan gum, guar gum, locust bean gum, poly vinyl acetate, and polyvinyl alcohol.

3. The timed release pharmaceutical composition according to claim 1 wherein the hydrophobic release controlling agent(s) is selected from hydrogenated vegetable oil, purified grades of beeswax, fatty acids, long chain fatty alcohols, glycerides, oils, acetylated glycerides, ethyl cellulose, stearic acid, paraffin, carnuba wax, talc, and stearate salts.

4. The time release pharmaceutical composition according to claim 1 wherein the natural release agent(s) is selected from hyaluronic acid, polyhyaluronic acid, alginic acid and sodium alginate.

5. The timed release pharmaceutical composition according to claim 1 wherein the synthetic release controlling agent(s) is selected from polyamides, polycarbonates, polyalkylenes, polyalkylene glycols, polyalkylene oxides, polyethylene oxide, polyalkylene terephthalates, polyvinyl alcohols, polyvinylphenol, polyvinyl ethers, polyvinyl esters, polyvinyl halides, polyvinylpyrrolidone, polyglycolides, polysiloxanes, polyure thanes, polystyrene, polylactides, poly (butyric acid), poly (valeric acid), poly(lactide-co-glycolide), poly (ethyleneterephthalate), poly (lactide-co- caprolactone), polyanhydrides, polyorthoesters, poly(fumaric acid), poly(maleic acid), polyvinyl acetate, polystyrene, polymers of acrylic and methacrylic esters (Eudragit® RSPO, Eudragit® RLPO, and Eudragit® L100-55), carbomer (carbopol®), celluloses and cellulose derivatives.

6. The timed release pharmaceutical composition according to claim 5 wherein the cellulose derivative is selected from methyl cellulose, ethyl cellulose, hydroxypropyl cellulose, hydroxypropylmethyl cellulose, hydroxybutylmethyl cellulose, hydroxyl ethyl cellulose, sodium carboxymethyl cellulose, cellulose acetate, cellulose propionate, cellulose acetate butyrate, cellulose acetate phthalate, carboxymethyl cellulose, cellulose triacetate, and cellulose sulfate sodium salt.

7. The timed release pharmaceutical composition according to claim 1, for use in the treatment of a patient suffering from a disease or a condition **characterized by** symptoms of dementia and/or cognitive impairments, wherein the said treatment administered orally provides a reduced incidence of insomnia and nightmares.

8. The timed release pharmaceutical composition according to claim 1, for use in the treatment of a patient suffering from a disease or a condition **characterized by** symptoms of dementia and/or cognitive impairments.

9. The timed release pharmaceutical composition according to claim 1, for use in the treatment of a patient suffering from mild, moderate or severe Alzheimer's disease.

## Patentansprüche

1. Pharmazeutische Zusammensetzung mit zeitgesteuerter Freigabe, umfassend Donepezil oder ein pharmazeutisches Salz davon, wobei die Zusammensetzung eine beschichtete Zusammensetzung ist, die Folgendes umfasst:
a) einen Kern, umfassend Donepezil in einer Menge von 3 mg bis 50 mg und ein oder mehrere Freigabesteuerungsmittel, und
b) eine funktionelle Beschichtung, umfassend ein oder mehrere Freigabesteuerungsmittel;
wobei das/die Freigabesteuerungsmittel ausgewählt sind ist/sind aus hydrophilen Freigabesteuerungsmitteln, hydrophoben Freigabesteuerungsmitteln, natürlichen Freigabesteuerungsmitteln und synthetischen Freigabesteuerungsmitteln;
die Menge von Freigabesteuerungsmittel in der Zusammensetzung 10 Gew.-% bis 50 Gew.-% des Gesamtgewichts der Zusammensetzung beträgt; und
die Zusammensetzung das folgende in-vitro-Auflösungsprofil aufweist, wenn sie in einer Paddle-Auflösungsvorrichtung bei 50 rpm in 900 ml Puffer mit pH-Wert 6,8 bei 37 °C untersucht wird, weniger als 20 Gew.-% Donepezil werden in 3-4 Stunden freigegeben und mehr als 85 Gew.-% Donepezil werden nach 12 Stunden freigegeben.

2. Pharmazeutische Zusammensetzung mit zeitgesteuerter Freigabe nach Anspruch 1, wobei das/die hydrophile Freigabesteuerungsmittel ausgewählt ist aus Hydroxyethylcellulose, Hydroxypropylcellulose, Hydroxypropylmethylcellulose, Natriumcarboxymethylcellulose, Natriumalginat, Carbomer, Xanthangummi, Guargummi, Johannisbrotkernmehl, Polyvinylacetat und Polyvinylalkohol.

3. Pharmazeutische Zusammensetzung mit zeitgesteuerter Freigabe nach Anspruch 1, wobei das/die hydrophobe Freigabesteuerungsmittel ausgewählt ist aus hydriertem Pflanzenöl, gereinigter Qualität von Bienenwachs, Fettsäuren, langkettigen Fettalkoholen, Glyceriden, Ölen, acetylierten Glyceriden, Ethylcellulose, Stearinsäure, Paraffin, Carnaubawachs, Talk und Stearatsalzen.

4. Pharmazeutische Zusammensetzung mit zeitgesteuerter Freigabe nach Anspruch 1, wobei das/die natürliche Freigabesteuerungsmittel ausgewählt ist aus Hyaluronsäure, Polyhyaluronsäure, Alginsäure und Natriumalginat.

5. Pharmazeutische Zusammensetzung mit zeitgesteuerter Freigabe nach Anspruch 1, wobei das/die synthetische Freigabesteuerungsmittel ausgewählt ist aus Polyamiden, Polycarbonaten, Polyalkylenen, Polyalkylenglykolen, Polyalkylenoxiden, Polyethylenoxiden, Polyalkylenterephthalaten, Polyvinylalkoholen, Polyvinylphenol, Polyvinylethern, Polyvinylestern, Polyvinylhaliden, Polyvinylpyrrolidon, Polyglycoliden, Polysiloxanen, Polyurethanen, Polystyrol, Polylactiden, Poly(buttersäure), Poly(valeriansäure), Poly(lactid-co-glycolid), Poly(ethylenterephthalat), Poly(lactid-co-caprolacton), Polyanhydriden, Polyorthoestern, Poly(fumarsäure), Poly(maleinsäure), Polyvinylacetat, Polystyrol, Polymeren von Acryl- und Methacrylestern (Eudragit® RSPO, Eudragit® RLPO und Eudragit® L100-55), Carbomer (Carbopol®), Cellulosen und Cellulosederivaten.

6. Pharmazeutische Zusammensetzung mit zeitgesteuerter Freigabe nach Anspruch 5, wobei das Cellulosederivat ausgewählt ist aus Methylcellulose, Ethylcellulose, Hydroxypropylcellulose, Hydroxypropylmethylcellulose, Hydroxybutylmethylcellulose, Hydroxyethylcellulose, Natriumcarboxymethylcellulose, Celluloseacetat, Cellulosepropionat, Celluloseacetatbutyrat, Celluloseacetatphthalat, Carboxymethylcellulose, Cellulosetriacetat und Cellulosesulfatnatriumsalz.

7. Pharmazeutische Zusammensetzung mit zeitgesteuerter Freigabe nach Anspruch 1 zur Verwendung bei der Behandlung eines Patienten, der unter einer Krankheit oder einem Gesundheitszustand leidet, **gekennzeichnet durch** Symptome von Demenz und/oder kognitiven Beeinträchtigungen, wobei die oral verabreichte Behandlung ein verringertes Vorkommen von Schlaflosigkeit und Albträumen bereitstellt.

8. Pharmazeutische Zusammensetzung mit zeitgesteuerter Freigabe nach Anspruch 1 zur Verwendung bei der Behandlung eines Patienten, der unter einer Krankheit oder einem Gesundheitszustand leidet, **gekennzeichnet durch** Symptome von Demenz und/oder kognitiven Beeinträchtigungen.

9. Pharmazeutische Zusammensetzung mit zeitgesteuerter Freigabe nach Anspruch 1 zur Verwendung bei der Behandlung eines Patienten, der unter leichter, mittelschwerer oder schwerer Alzheimer-Krankheit leidet.

## Revendications

1. Composition pharmaceutique à libération prolongée, comprenant du donépézil ou un sel pharmaceutiquement acceptable de celui-ci, dans laquelle la composition est une composition enrobée, qui comprend :
a) un noyau comprenant du donépézil en une quantité de 3 mg à 50 mg, et un ou plusieurs agent(s) de prolongation de libération, et
b) un enrobage fonctionnel comprenant un ou plusieurs agent(s) de prolongation de libération ;
dans laquelle l'agent ou les agents de prolongation de libération est/sont sélectionné(s) parmi des agents de prolongation de libération hydrophiles, des agents de prolongation de libération hydrophobes, des agents de prolongation de libération naturels et des agents de prolongation de libération synthétiques ;
la quantité d'agent de prolongation de libération dans la composition est de 10 % m/m à 50 % m/m en poids total de la composition ; et
la composition présente le profil de dissolution in vitro suivant lorsqu'il fait l'objet d'essais dans un appareil de dissolution Paddle à 50 tr/min dans 900 ml de tampon de pH 6,8 à 37°C : moins de 20 % m/m de donépézil est libéré au cours de 3 à 4 h, et plus de 85 % m/m de donépézil est libéré après 12 h.

2. Composition pharmaceutique à libération prolongée selon la revendication 1, dans laquelle l'agent ou les agents de prolongation de libération hydrophile(s) est/sont sélectionné(s) parmi : hydroxyéthylcellulose, hydroxypropylcellulose, hydroxypropylméthylcellulose, carboxyméthylcellulose de sodium, alginate de sodium, carbomère, gomme de xanthane, gomme de guar, gomme de caroube, acétate polyvinylique, et alcool polyvinylique.

3. Composition pharmaceutique à libération prolongée selon la revendication 1, dans laquelle l'agent ou les agents de prolongation de libération hydrophobes est/sont sélectionné(s) parmi : huile végétale hydrogénée, classes purifiées de cire d'abeille, acides gras, alcools gras à chaîne longue, glycérides, huiles, glycérides acétylés, éthylcellulose, acide stéarique, paraffine, cire de carnauba, talc, et sels de stéarate.

4. Composition pharmaceutique à libération prolongée selon la revendication 1, dans laquelle l'agent ou les agents de libération naturel(s) est/sont sélectionné(s) parmi : acide hyaluronique, acide polyhyaluronique, acide alginique et alginate de sodium.

5. Composition pharmaceutique à libération prolongée selon la revendication 1 dans laquelle l'agent ou les agents de prolongation de libération synthétique(s) est/sont sélectionné(s) parmi : polyamides, polycarbonates, polyalkylènes, polyalkylène glycols, oxydes de polyalkylène, oxyde de polyéthylène, polyalkylène téréphtalates, alcools polyvinyliques, polyvinylphénol, éthers polyvinyliques, esters polyvinyliques, halogénures polyvinyliques, polyvinylpyrrolidone, polyglycolides, polysiloxanes, polyuréthanes, polystyrène, polylactides, poly(acide butyrique), poly(acide valérique), poly(lactide-co-glycolide), poly(éthylène téréphtalate), poly(lactide-co-caprolactone), polyanhydrides, polyorthoesters, poly(acide fumarique), poly(acide maléique), acétate de polyvinyle, polystyrène, polymères d'esters acryliques et méthacrylique (Eudragit® RSPO, Eudragit® RLPO, et Eudragit® L100-55), carbomère (carbopol®), celluloses et dérivés de cellulose.

6. Composition pharmaceutique à libération prolongée selon la revendication 5, dans laquelle le dérivé de cellulose est sélectionné parmi : méthylcellulose, éthylcellulose, hydroxypropylcellulose, hydroxypropylméthylcellulose, hydroxybutylméthyl cellulose, hydroxyléthylcellulose, carboxyméthylcellulose de sodium, acétate de cellulose, propionate de cellulose, acétate butyrate de cellulose, acétate phtalate de cellulose, carboxyméthylcellulose, triacétate de cellulose, et sel sodique de sulfate de cellulose.

7. Composition pharmaceutique à libération prolongée selon la revendication 1, pour l'utilisation dans le traitement d'un patient souffrant d'une maladie ou d'une condition **caractérisée par** des symptômes de démence et/ou de troubles cognitifs, dans laquelle ledit traitement administré oralement fournit une incidence réduite d'insomnie et de cauchemar.

8. Composition pharmaceutique à libération prolongée selon la revendication 1, pour l'utilisation dans le traitement d'un patient souffrant d'une maladie ou d'une condition **caractérisée par** des symptômes de démence et/ou de troubles cognitifs.

9. Composition pharmaceutique à libération prolongée selon la revendication 1, pour l'utilisation dans le traitement d'un patient souffrant de maladie d'Alzheimer de stade léger, modéré ou sévère.
